# EUROPÄISCHE PATENTSCHRIFT

(11) **EP 0 211 311 B1**
(45) Veröffentlichungstag und Bekanntmachung des Hinweises auf die Patenterteilung: **05.05.1993**
(21) Anmeldenummer: 86110030.3
(22) Anmeldetag: 22.07.1986
(51) Int. Cl.: A61L 15/16, A61L 15/00, C09J 147/00

(54) **Selbstklebender absorptionsfähiger Formkörper und Verfahren zum Herstellen eines dafür geeigneten Haftschmelzklebers**
Self-adhesive absorbing article and method for the preparation of a hot-melt adhesive therefor
Article absorbant auto-adhésif et procédé de préparation d'un adhésif thermofusible approprié

(30) Priorität: 07.08.1985 DE 3528355
(43) Veröffentlichungstag der Anmeldung: 25.02.1987
(73) Patentinhaber: H.B. FULLER LICENSING & FINANCING, INC., Wilmington, Delaware 19801 (US)
(72) Erfinder: Janssen, Annegret, D-2120 Lüneburg (DE)
(74) Vertreter: Maiwald, Walter, Dr. Dipl.-Chem.

(56) Entgegenhaltungen:
- DE-U- 8 522 762
- US-A- 4 210 570
- US-A- 4 264 756
- US-A- 4 460 728

## Beschreibung

Die Erfindung betrifft einen absorptionsfähigen Formkörper, beispielsweise Damenbinde oder Slipeinlage, mit einem Kern aus absorbierendem Material und einer Hülle aus flüssigkeitsdurchlässigem Blattmaterial, wobei auf einer Seite des Formkörpers auf der Oberfläche der Hülle eine druckempfindliche selbstklebende Klebstoffschicht aus Haftschmelzkleber, die mit einer ablösbaren Schutzfolie abgedeckt ist, vorgesehen ist. Außerdem betrifft die Erfindung ein Verfahren zumHerstellen eines für die selbstklebende Klebstoffschicht dieses Formkörpers geeigneten Haftschmelzklebers.

Es ist bekannt, absorptionsfähige Formkörper wie Damenbinden, Slipeinlagen und sonstige Hygieneartikel mit einer selbstklebenden Klebstoffschicht aus Haftschmelzkleber auszurüsten, um den Formkörper in einem Bekleidungsstück wie beispielsweise einem Slip anbringen und befestigen zu können. Dabei ist einerseits eine einwandfreie Haftung für einen sicheren Sitz des Formkörpers notwendig sowie ein rückstandsfreies Ablösen desselben vom Stoff des Bekleidungs- bzw. Wäschestückes.

Für selbstklebende Etiketten, Klebebänder, Teppichfliesen, Hygieneartikel und andere Artikel mit selbstklebender Ausrüstung verwendet man Haftschmelzkleber, die hauptsächlich aus Polymeren, Weichmacherölen, klebrig machenden Harzen und Zusatzstoffen bestehen. Als Polymer wird thermoplastischer Kautschuk wie SEBS, SBS, SBR, SIS allein oder in Kombination miteinander eingesetzt, in Einzelfällen auch EVA. Die Polymerkomponente des Haftschmelzklebers beeinflußt primär die Kohäsion des Haftschmelzklebers, während die Harzkomponente primär die adhäsiven Kräfte bestimmt.

Eine Besonderheit von Haftschmelzklebern im Vergleich zu anderen Schmelzklebstoffen ist, daß sie eine rein adhäsive Verklebung ermöglichen. Entsprechend den unterschiedlichen Anwendungen und den daraus resultierenden Anforderungen an die Gebrauchseignung sind Kohäsion und Adhäsion des jeweiligen Haftschmelzklebers unterschiedlich ausgeprägt. Ihr Verhältnis ist besonders in den Fällen kritisch, in denen die Verklebung wieder lösbar sein soll, ohne die verklebten Substrate zu zerstören und ohne an dem nicht mit dem Haftschmelzkleber beschichteten Substrat Klebstoffrückstände zu hinterlassen.

Dies ist besonders wichtig für Hygieneartikel wie Damenbinden, Slipeinlagen und Inkontinenzartikel, bei denen an die Haftschmelzkleber besondere Anforderungen gestellt werden, denn sie müssen einerseits ausreichende Adhäsion für gute Haftung und sicheren Sitz aufweisen, wobei die Adhäsion aber die Festigkeit irgendeines Teiles des Hygieneartikels nicht übersteigen darf, damit der Hygieneartikel unversehrt wieder entfernt werden kann, und gleichzeitig eine ausreichende Kohäsion haben, um ein einfaches und rückstandsfreies Ablösen des Hygieneartikels vom Wäschestück zu gewährleisten. Es darf also durch das Ankleben des Hygieneartikels am Wäschestück kein Klebstoffübergang stattfinden.

Die Adhäsion eines Haftschmelzklebers wird anhand der Schälfestigkeit bestimmt, die Kohäsion anhand der Scherfestigkeit. Für Hygieneartikel erscheinende bekannte Haftschmelzkleber haben - nach üblichen Normprüfmethoden von PSTC und FINAT ermittelt - einen Adhäsionsbereich mit Schälfestigkeitswerten zwischen 9 und 40 N/25 mm. Haftschmelzkleber mit thermoplastischem Kautschuk als Polymerbasis haben generell eine hohe Kohäsion mit Scherfestigkeitswerten von mehr als 2 h. Ebenfalls sind Haftschmelzkleber auf EVA-Basis mit gleichen Adhäsionseigenschaften, aber niedriger Kohäsion mit Scherfestigkeitswerten um 5 min.,die sich rückstandsfrei lösen lassen, bekannt. Solche bisher für selbstklebend ausgerüstete Hygieneartikel verwendeten Haftschmelzkleber sind jedoch wegen der genannten Polymerbasis - Zusammensetzung verhältnismäßig kostspielig.

Das amerikanische Patent 4 210 570 offenbart die Verarbeitung bestimmter amorpher oder teilkristalliner Olefincopolymere mit bestimmten Harzen und Weichmacherölen zu Haftklebern. Die olefinischen Copolymere sollen dabei wenigstens ein lineares C₃-C₅-α-Olefin und 15-60 mol % wenigstens eines linearen C₆-C₁₀-α-Olefins enthalten, wobei offen bleibt, ob es sich hierbei um ein ataktisches Polymer handelt. Insbesondere wird die hohe Scherfestigkeit der offenbarten Haftschmelzkleber hervorgehoben.

Dieser Stand der Technik lehrt, die bekannten Haftschmelzkleber so auszugestalten, daß sie Scherfestigkeiten von wenigstens 19 Minuten, weit überwiegend aber von zwischen 1000 und oberhalb 10.000 Minuten haben. Damit wird die Meinung bekräftigt, daß Haftschmelzkleber hohe Scherfestigkeiten benötigen.

Noch höhere Scherfestigkeiten zeigen die Haftschmelzkleber gemäß US-Patent 4 264 756, bei denen im Unterschied zum vorstehend genannten Stand der Technik kein Weichmacheröl vorgesehen wird.

Die Aufgabe der Erfindung besteht darin, einen selbstklebenden absorptionsfähigen Formkörper mit preiswerter als bisher möglich erscheinender Haftschmelzkleberbeschichtung bereit zu stellen, der mit ausreichender Festigkeit sicher in einem Wäschestück zu befestigen und von diesem auch zerstörungsfrei und rückstandsfrei wieder zu lösen ist.

Diese Aufgabe wird erfindungsgemäß mit einem absorptionsfähigen Formkörper gelöst, der die Merkmale des Patentanspruches 1 aufweist. Vorteilhafte Ausgestaltungen dieses Formkörpers sind Gegenstand der Ansprüche 2 bis 4.

Die Ansprüche 5 bis 8 definieren einen erfindungsgemäßen Haftschmelzkleber sowie dessen vorteilhafte ausgestaltungen.

Der erfindungsgemäß benutzte Haftschmelzkleber enthält als Polymerbasis ataktische Copolymere, und zwar entweder allein oder in Gemisch mit ataktischem Polypropylen, und ferner die bei bekannten Haftschmelzklebern üblichen Zusatzstoffe. Die Adhäsionswerte derartiger Haftschmelzkleber liegen im üblichen Rahmen, ihre Kohäsionswerte dagegen sehr niedrig. Haftschmelzkleber entsprechend der vorliegenden Erfindung lassen sich unter den üblichen Normprüfbedingungen (Haftungs- und Ablöseverhalten gegenüber Stahl) nicht rückstandsfrei wieder ablösen. Es wurde jedoch gefunden, daß derartige Haftschmelzkleber gleichwohl die erforderliche Gebrauchseignung für Hygieneartikel aufweisen. Diese Haftschmelzkleber lassen sich preiswerter als für Hygieneartikel bisher als geeignet gehaltene Haftschmelzkleber herstellen, so daß sich die Kosten entsprechend ausgerüsteter absorptionsfähiger Formkörper merklich senken lassen, weil der verwendete Haftschmelzkleber ein nicht unwesentlicher Kostenfaktor derartiger Formkörper ist.

Die Gebrauchseignung der erfindungsgemäß eingesetzten Haftschmelzkleber für Hygieneartikel wurde abweichend von den üblichen Normprüfmethoden auf folgende Weise ermittelt:

Es wurden die Klebeigenschaften einer Standardbeschichtung (60 g/m² Haftschmelzkleber auf Polyester) gegen Baumwollstoff bei Raumtemperatur (RT) und nach simulierten Gebrauchsbedingungen (TTP = Temperatur 38°C, Time = Zeit 15 h, Pressure = Druck 0,13 N/cm²) ermittelt. Dabei zeigte sich für bisher eingesetzte Haftschmelzkleber Schälfestigkeitswerte in folgenden Spannen:

| | |
|---|---|
| RT | 0,1 - 2,5 N/25 mm |
| TTP | 0,7 - 4,0 N/25 mm |

In diesem Rahmen liegen auch die Werte der erfindungsgemäß eingesetzten Haftschmelzkleber, die die für den sicheren Sitz des Formkörpers am Wäschestück erforderliche Adhäsion aufweisen, sich zugleich aber einfach und rückstandsfrei vom Wäschestoff lösen lassen. Folgende Schälfestigkeiten wurden für einen derartigen Haftschmelzkleber ermittelt:
Standardprüfung der Anmelderin gegen Baumwolle:

| | | |
|---|---|---|
| Schälfestigkeit | RT | ca. 0,6 N/25 mm |
| | TTP | ca. 1,8 N/25 mm |

In beiden Fällen Ablösen von der Baumwolle ohne Rückstände
Zur weiteren Erläuterung der Erfindung dient das nachfolgende Beispiel eines Haftschmelzklebers auf Basis ataktischer Polymere:

### Rezeptur

30 Teile Mineralöl
33 Teile ataktisches Copolymerisat von alpha-Olefinen, Schmelzviskosität bei 190°C ca. 50.000 mPa.s, Ring+Kugel-Erweichungspunkt ca. 110°C, Penetration ca. 14
35 Teile Cyclopentadiene-Harz, Ring+Kugel-Erw. punkt ca. 125°C
2 Teile EVA
Würde ein solcher Haftschmelzkleber nach der Normprüfung gegen Stahl bewertet, würde er aufgrund der ermittelten Scherfestigkeit als ungeeignet beurteilt.

### Normprüfung gegen Stahl:

| | |
|---|---|
| Schälfestigkeit | 28 N/25 mm, Kohäsionsbruch beim Ablösen, d.h. Klebstoffrückstände |
| Scherfestigkeit | < 1 Min. |

### Rezepturspannen der Grund-Rohstoffe:

| | |
|---|---|
| ataktisches Polymer | 20 - 80 % |
| Harz | 20 - 80 % |
| Öl | 0 - 80 % |

Es wurde gefunden, daß für Formkörper wie Hygieneartikel Haftschmelzkleber völlig ausreichend sind, deren Kohäsion gegenüber Stahl nicht ausreicht. Die Schälfestigkeit ist gegenüber Baumwolle und nicht gegenüber Stahl zu ermitteln, um feststellen zu können, ob ein Haftschmelzkleber für Hygieneartikel geeignet ist. Auf diese Weise wird man in die Lage versetzt, auch billigere Haftschmelzkleber auf deren Eignung für Hygieneartikel zu untersuchen.

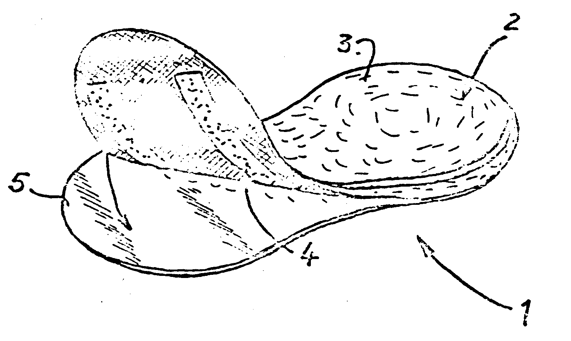

Zur weiteren Erläuterung der Erfindung ist in der Zeichnung schematisch und schaubildlich ein erfindungsgemäß ausgestalteter absorptionsfähiger Formkörper dargestellt.

Der in der Zeichnung gezeigte Formkörper (1) hat einen länglichen Absorptionskörper (2) mit abgerundeten Enden und etwas nach einwärts gehenden Seitenkanten, jedoch ist die Form des Absorptionskörpers für die vorliegende Erfindung nicht entscheidend. Der Absorptionskörper (2) hat eine flüssigkeitsdurchlässige äußere Hülle (3) und einen darin untergebrachten, in der Zeichnung nicht dargestellten Kern aus flüssigkeitsabsorbierendem Material .

Auf der in der Zeichnung unten liegenden Seite des Absorptionskörpers (2) sind sich über ein Großteil der Länge desselben erstreckende Streifen (4) aus erfindungsgemäßem Haftschmelzkleber vorgesehen, der auf der Basis ataktischer Polymere gebildet ist. Diese mit den Haftschmelzkleber-Streifen (4) versehene Seite des Absorptionskörper (3) ist mittels einer dünnen abziehbaren Schutzfolie (5) vor Gebrauch abgedeckt. Diese Schutzfolie (5) ist in der Zeichnung teilweise vom Absorptionskörper (3) getrennt dargestellt und besteht beispielsweise aus Silikonpapier. Unmittelbar vor Ingebrauchnahme des Formkörpers (1) wird sie von diesem abgezogen.

Obwohl die Haftschmelzkleber-Beschichtung in der Zeichnung in Form von Streifen (4) dargestellt ist, kann sie auch vollflächig und über die gesamte Länge des Absorptionskörpers (2) ausgebildet sein oder auch beliebige andere Muster wie das Streifenmuster aufweisen.

## Patentansprüche

1. Absorptionsfähiger Formkörper, beispielsweise Damenbinde oder Slipeinlage, mit einem Kern aus absorbierendem Material und einer Hülle aus flüssigkeitsdurchlässigem Blattmaterial, wobei auf einer Seite des Formkörpers auf der Oberfläche der Hülle eine mit einer ablösbaren Schutzfolie abgedeckte druckempfindliche selbstklebende Klebstoffschicht vorgesehen ist, die aus einem Haftschmelzkleber aus 20 bis 80% im wesentlichen amorphen olefinischen Polymeren, 20 bis 80% klebrig machenden Harzen und 0 bis 80% Weichmacheröl besteht,
dadurch gekennzeichnet,
daß der Polymergehalt des Haftschmelzklebers aus ataktischen Polymeren besteht und der Haftschmelzkleber eine Scherfestigkeit (Normprüfung gegen Stahl) von weniger als 1 Minute aufweist.

2. Formkörper nach Anspruch 1,
dadurch gekennzeichnet,
daß die ataktischen Polymere ataktische Copolymere sind.

3. Formkörper nach Anspruch 2,
dadurch gekennzeichnet,
daß die ataktischen Copolymere mit ataktischem Polypropylen vermischt sind.

4. Formkörper nach einem der Ansprüche 1 bis 3,
dadurch gekennzeichnet,
daß das ataktische Polymere ein ataktisches Copolymerisat von alpha-Olefinen ist.

5. Haftschmelzkleber, bestehend aus 20 bis 80% im wesentlichen amorphen olefinischen Polymeren, 20 bis 80% klebrig machenden Harzen und 0 bis 80% Weichmacheröl,
dadurch gekennzeichnet,
daß die Polymeren ataktisch sind und der Haftschmelzkleber eine Scherfestigkeit (Normprüfung gegen Stahl) von weniger als 1 Minute aufweist.

6. Haftschmelzkleber nach Anspruch 5,
dadurch gekennzeichnet,
daß die ataktischen Polymeren ataktische Copolymere sind.

7. Haftschmelzkleber nach Anspruch 6,
dadurch gekennzeichnet,
daß die ataktischen Copolymeren mit ataktischem Polypropylen vermischt sind.

8. Haftschmelzkleber nach einem der Ansprüche 5 bis 7,
dadurch gekennzeichnet,
daß das ataktsiche Polymere ein ataktisches Copolymerisat von alpha-Olefinenen ist.

## Claims

1. Absorbing article for example a ladies' sanitary towel or pantliner, having a core of absorbing material and a sleeve comprising a sheet of liquid-permeable material, wherein a pressure-sensitive, self-adhesive adhesive layer which is covered by a removable protective film is provided on one side of the article on the surface of the sleeve, the said adhesive layer comprises a bonding hot-melt adhesive of 20 to 80% substantially amorphous olefinic polymers, 20 to 80% tackifying resins and 0 to 80% softening oil, characterised in that the polymer content of the bonding hot-melt adhesive comprising atactic polymers and the bonding hot-melt adhesive has a shearing strength (standard test against steel) of less than 1 minute.

2. Article according to claim 1, characterized in that the atactic polymers are atactic copolymers.

3. Article according to claim 2, characterised in that the atactic copolymers are mixed with atactic polypropylene.

4. Article according to any of claims 1 to 3, characterised in that the atactic polymer is an atactic copolymerisate of alpha-olefins.

5. Bonding hot-melt adhesive comprising 20 to 80% substantially amorphous olefinic polymers, 20 to 80% tackifying resins and 0 to 80% softening oil, characterised in that the polymers are atactic and the bonding hot-melt adhesive comprises a shearing strength (standard test against steel) of less than 1 minute.

6. Bonding hot-melt adhesive according to claim 5, characterized in that the atactic polymers are atactic copolymers.

7. Bonding hot-melt adhesive according to claim 6, characterized in that the atactic copolymers are mixed with atactic polypropylene.

8. Bonding hot-melt adhesive according to any of claims 5 to 7, characterised in that the atactic polymer is an atactic copolymerisate of alpha-olefins.

## Revendications

1. Corps moulé absorbant, par exemple serviette hygiénique ou protège-slip, comportant une partie centrale en matériau absorbant et une enveloppe en matériau en feuilles perméable aux liquides, sur un des côtés du corps moulé étant disposeé, sur la surface de l'enveloppe, une couche d'adhésif autocollant sensible à la pression, revêtue d'une pellicule protectrice détachable, qui est constituée d'un adhésif fusible de contact à base de 20 à 80 % de polymères oléfiniques pratiquement amorphes, de 20 à 80 % de résines rendant collant et de 0 à 80 % d'huile plastifiante, caractérisé en ce que la fraction polymère de l'adhésif fusible de contact consiste en polymères atactiques et l'adhésif fusible de contact présente une résistance au cisaillement (essai normalisé vis-à-vis de l'acier) de moins de 1 minute.

2. Corps moulé selon la revendication 1, caractérisé en ce que les polymères atactiques sont des copolymères atactiques.

3. Corps moulé selon la revendication 2, caractérisé en ce que les copolymères atactiques sont mélangés avec du polypropylène atactique.

4. Corps moulé selon l'une des revendications 1 à 3, caractérisé en ce que le polymère atactique est un copolymère atactique d'α-oléfines.

5. Adhésif fusible de contact constitué de 20 à 80 % de polymères oléfiniques pratiquément amorphes, de 20 à 80 % de résines rendant collant et de 0 à 80 % d'huile plastifiante, caractérisé en ce que les polymères sont atactiques et l'adhésif fusible de contact présente une résistance au cisaillement (essai normalisé vis-à-vis de l'acier) de moins de 1 minute.

6. Adhésif fusible de contact selon la revendication 5, caractérisé en ce que les polymères atactiques sont des copolymères atactiques.

7. Adhésif fusible de contact selon la revendication 6, caractérisé en ce que les copolymères atactiques sont mélangés avec du polypropylène atactique.

8. Adhésif fusible de contact selon l'une des revenditions 5 à 7, caractérisé en ce que le polymère atactique est un copolymère atactique d'α-oléfines.
